# EUROPÄISCHE PATENTSCHRIFT

(11) **EP 2 919 652 B1**
(45) Veröffentlichungstag und Bekanntmachung des Hinweises auf die Patenterteilung: **24.06.2020**
(21) Anmeldenummer: 13779520.9
(22) Anmeldetag: 10.10.2013
(51) Int. Cl.: A61B 5/12, H04R 25/00, A61M 21/00

(54) **BEARBEITEN VON AUDIOSIGNALEN FÜR EINE TINNITUSTHERAPIE**
PROCESSING OF AUDIO SIGNALS FOR A TINNITUS THERAPY
TRAITEMENT DE SIGNAUX AUDIO POUR LA THÉRAPIE DES ACOUPHÈNES

(30) Priorität: 13.11.2012 DE 102012220620
(43) Veröffentlichungstag der Anmeldung: 23.09.2015
(73) Patentinhaber: Sonormed GmbH, 20357 Hamburg (DE)
(72) Erfinder: NÖTZEL, Marc Adrian, 20357 Hamburg (DE); WITTIG, Johannes Abraxas, 38118 Braunschweig (DE); LAND, Jörg, 20357 Hamburg (DE); LANZ, Matthias, 22523 Hamburg (DE)
(74) Vertreter: Seemann & Partner Patentanwälte mbB
(86) Internationale Anmeldenummer: PCT/EP2013/003042
(87) Internationale Veröffentlichungsnummer: WO 2014/075753

(56) Entgegenhaltungen:
- WO-A1-2011/127930
- WO-A2-2008/087157
- DE-A1-102011 001 793

## Beschreibung

Die Erfindung betrifft ein Verfahren zum Bearbeiten von Audiosignalen für eine Therapie eines subjektiven Tinnitus mit einer individuellen Tinnitusfrequenz. Die Erfindung betrifft des Weiteren ein Computerprogramm.

Die Wahrnehmung von Geräuschen ohne die Existenz einer internen oder externen Geräuschquelle wird als subjektiver Tinnitus bezeichnet. Ein Tinnitus ist eine oft chronische Erkrankung und tritt in der Regel mit einer konstanten individuellen Tinnitusfrequenz auf. Als physiologische Ursache hierfür liegt meist eine abnorme neuronale Aktivität in der primären Hörrinde vor.

Eine mögliche Therapie zur Linderung eines subjektiven Tinnitus beruht auf dem Ansatz, die abnorme neuronale Aktivität in der Hörrinde unter Ausnutzung lateraler Hemmung zu reduzieren und dadurch eine therapeutisch wirksame Normalisierung dieser neuronalen Aktivität aufgrund neuronaler Plastizität anzuregen.

Unter lateraler Hemmung wird dabei insbesondere eine charakteristische Verschaltung der Nervenzellen im zentralen Nervensystem verstanden, die bewirkt, dass bei einem peripheren Reiz bestimmte Nervenzellen stimuliert und die Aktivität anderer Nervenzellen für die Wahrnehmung vergleichbarer Reize gehemmt wird.

Die Therapie besteht entsprechend darin, Geräusche oder Musik zu hören, bei der die Frequenzanteile im Bereich der Tinnitusfrequenz herausgefiltert wurden. Beispielsweise wird hierfür weißes Rauschen verwendet, aus dem für beliebige Tinnitusfrequenzen Therapiedaten erzeugt werden können, weil weißes Rauschen ein sehr breites und gleichmäßiges Frequenzspektrum aufweist. Weißes Rauschen wird jedoch auf Dauer von den Patienten als störend und unangenehm empfunden. Dadurch sinkt die Bereitschaft, die Therapie regelmäßig und dauerhaft anzuwenden.

Angenehmer zu hören ist Musik, wobei bisher nur Musik von besonders hoher Audioqualität mit professioneller Aufbereitung als geeignet betrachtet wird. Da zudem eine individuelle Bearbeitung für jeden einzelnen Patienten erforderlich ist, stehen einem Patienten in der Regel nur wenige verschiedene Musikstücke für die Therapie zur Verfügung. Diese entsprechen zudem oft nicht den persönlichen Vorlieben des Patienten.

DE 10 2011 001 793 A1 offenbart ein Hörgerät sowie ein Verfahren zum Einstellen und Betreiben eines Hörgerätes. Das Hörgerät weist einen Filter auf, der dazu eingerichtet ist, ein Umgebungsschallsignal in einem vorbestimmten Frequenzbereich abzusenken oder zu sperren. Hierdurch soll für den Tinnituspatienten ein angenehmer Höreindruck erzeugt und die Sprachverständlichkeit gesteigert werden.

WO 2008/087157 A2 offenbart eine Vorrichtung zur Behandlung von Tinnitus, wobei ein mittels eines geeigneten Generators erzeugtes Audiosignal im Bereich einer Tinnitusfrequenz gefiltert wird. Hierbei ist das erzeugte Audiosignal beispielsweise weißes Rauschen.

WO 2011/127930 A1 offenbart ein Hörgerät und ein Verfahren zur Milderung eines Tinnitus. Das Hörgerät umfasst einen zuschaltbaren Bandfilter und erlaubt so eine Tinnitustherapie mit individuell als angenehm empfundener Musik.

Ausgehend von diesem Stand der Technik besteht die Aufgabe der vorliegenden Erfindung darin, die Verfügbarkeit von für die Tinnitustherapie geeigneten Audiodaten zu verbessern und insbesondere eine Tinnitustherapie basierend auf nach persönlichen Vorlieben ausgewählten Audiodaten zu ermöglichen.

Diese Aufgabe wird gelöst durch ein Verfahren zum Bearbeiten von Audiosignalen, insbesondere für eine Therapie eines subjektiven Tinnitus mit einer individuellen Tinnitusfrequenz, umfassend die folgenden Verfahrensschritte:
- Bereitstellen eines ersten Audiosignals,
- Bestimmen eines Sperrbereichs im Frequenzspektrum des ersten Audiosignals mit einer vorgebbaren Frequenzbreite anhand einer vorgebbaren Therapiefrequenz, wobei der Filter ein Bandsperrfilter ist, durch den Signalanteile des ersten Audiosignals mit Frequenzen im Sperrbereich beim Erstellen des zweiten Audiosignals ganz oder teilweise entfernt werden,
- Erstellen eines zweiten Audiosignals aus dem ersten Audiosignal unter Verwendung eines Filters für einen Signalanteil des ersten Audiosignals im Sperrbereich,

- Ermitteln einer auditiven Energie des ersten Audiosignals oder des zweiten Audiosignals innerhalb wenigstens eines vorgegebenen oder vorgebbaren therapeutisch verwendbaren Frequenzbereichs, und
- Bestimmen eines Bewertungsparameters zu dem zweiten Audiosignal in Abhängigkeit von der auditiven Energie und von einem Frequenzabstand zwischen dem therapeutisch verwendbaren Frequenzbereich und dem Sperrbereich, wobei der Bewertungsparameter geeignet definiert ist, um anzugeben, wie stark die Aktivität der zu der Therapiefrequenz oder zum Sperrbereich tonotopen Neuronen durch laterale Hemmung aufgrund der Stimulierung von zum therapeutisch verwendbaren Frequenzbereich tonotopen Neuronen beim Hören des zweiten Audiosignals gehemmt wird.

Ein Vorteil der Erfindung besteht darin, dass grundsätzlich beliebige Audiosignale als erstes Audiosignal in Frage kommen, wobei mittels des Bewertungsparameters ein objektiver Maßstab zur Verfügung gestellt wird, inwieweit das zweite Audiosignal zur Therapie des Tinnitus mit der individuellen Tinnitusfrequenz des vorliegenden Einzelfalls geeignet ist. Vorzugsweise wird das zweite Audiosignal nur dann zur Tinnitustherapie freigegeben oder verwendet, sofern der Bewertungsparameter über einem vorgebbaren Bewertungsparametergrenzwert liegt.

Der Bewertungsparameter ist geeignet definiert, um anzugeben, wie stark die Aktivität der zu der Therapiefrequenz bzw. zum Sperrbereich tonotopen Neuronen durch laterale Hemmung aufgrund der Stimulierung von zum therapeutisch verwendbaren Frequenzbereich tonotopen Neuronen beim Hören des zweiten Audiosignals gehemmt wird. Hierfür werden beispielsweise Kenntnisse und Modelle der Funktionsweise des menschlichen Gehörs, insbesondere der lateralen Hemmung zwischen den Neuronen der primären Hörrinde, berücksichtigt.

Damit ist der Bewertungsparameter insbesondere ein objektiver Maßstab dafür, wie stark die für den Tinnitus ursächliche abnorme Aktivität der Neuronen beim Hören des zweiten Audiosignals gehemmt wird. Da dies das Ziel der Tinnitustherapie ist, kann beispielsweise für vorhandene Audiosignale, die sich bei einer Tinnitustherapie empirisch als geeignet oder ungeeignet erwiesen haben, der jeweilige Bewertungsparameter bestimmt werden und durch Vergleich mit dem für das zweite Audiosignal ermittelten Bewertungsparameter angegeben werden, inwieweit das zweite Audiosignal zur Tinnitustherapie geeignet ist.

Ein weiterer Vorteil der Erfindung besteht darin, dass anhand des Bewertungsparameters eine gezielte Steuerung der Therapie ermöglicht wird. Beispielsweise kann der behandelnde Arzt unter Berücksichtigung des Bewertungsparameters vorgeben, wie oft oder wie lange das zweite Audiosignal für einen optimalen Therapieerfolg gehört werden soll.

Der erfindungsgemäß verwendete Filter ist ein Bandsperrfilter, durch den Signalanteile des ersten Audiosignals mit Frequenzen im Sperrbereich beim Erstellen des zweiten Audiosignals ganz oder teilweise entfernt werden. Die Wirkung des Bandsperrfilters soll dabei möglichst auf den Sperrbereich beschränkt sein, so dass insbesondere das erste Audiosignal und das zweite Audiosignal außerhalb des Sperrbereichs im Wesentlichen übereinstimmen.

Der therapeutisch verwendbare Frequenzbereich wird insbesondere derart vorgegeben, dass kein Überlapp mit dem Sperrbereich besteht. Die auditive Energie des ersten Audiosignals innerhalb des therapeutisch verwendbaren Frequenzbereichs unterscheidet sich daher im Wesentlichen nicht von derjenigen des zweiten Audiosignals.

Unter auditiver Energie wird insbesondere die über alle Frequenzen eines Frequenzbereichs oder eines Frequenzintervalls summierte oder integrierte Schallenergie eines Audiosignals verstanden. Unter einem Frequenzbereich oder Frequenzintervall kann auch eine Bandbreite verstanden werden. Die auditive Energie innerhalb eines Frequenzbereichs korreliert somit mit der Stärke einer Stimulierung der zu diesem Frequenzbereich tonotopen Neuronen.

Das erfindungsgemäße Verfahren ist vorzugsweise dadurch gekennzeichnet, dass das erste Audiosignal und/oder das zweite Audiosignal jeweils ein digitales Audiosignal, insbesondere eine digitale Audiodatei oder ein digitaler Audiodatenstrom, ist.

Unter einer digitalen Audiodatei ist insbesondere ein gespeichertes digitales Audiosignal zu verstehen, auf das wiederholt und zeitasynchron zugegriffen werden kann. Demgegenüber wird unter einem Audiodatenstrom insbesondere ein Audiosignal verstanden, das einmalig und/oder zeitsynchron oder in Echtzeit zur Verfügung steht.

Das erste Audiosignal wird vor dem Erstellen des zweiten Audiosignals vorzugsweise normalisiert. Hierdurch werden der Signal-Rausch-Abstand des zweiten Audiosignals verbessert und insbesondere Rauscheffekte, die beim Erstellen des zweiten Audiosignals, insbesondere beim Anwenden des Filters, auftreten, reduziert.

Unter Normalisieren oder Aussteuern wird im Rahmen der Erfindung insbesondere verstanden, dass das erste Audiosignal derart skaliert wird, dass der höchste Signalwert innerhalb des ersten Audiosignals einem vorgegebenen Maximalwert und/oder der niedrigste Signalwert innerhalb des ersten Audiosignals einem vorgebebenen Minimalwert entspricht. Im Fall digitaler Signale werden der Maximalwert und der Minimalwert beispielsweise durch die Quantisierungswortbreite des ersten Audiosignals bestimmt.

Unter Quantisierungswortbreite wird insbesondere in einem digitalen Audiosignal die Größe oder Wortlänge der digitalen Information für die Kodierung eines einzelnen Pulshöhenwertes verstanden. Bei einer Quantisierungswortbreite von 16 bit stehen beispielsweise 65536 verschiedene diskreten Werte für die Kodierung der Pulshöhe des Audiosignals zur Verfügung.

Vorzugsweise ist als weiterer Verfahrensschritt vorgesehen, dass das erste Audiosignal und/oder das zweite Audiosignal zum Ausgleich von frequenzabhängigen Überhöhungen und/oder Dämpfungen durch ein Abspielgerät mit nichtlinearem Frequenzgang korrigiert wird.

Bevorzugt wird das erste Audiosignal vor oder beim Erstellen des zweiten Audiosignals korrigiert. Die Korrektur kann auch beim zweiten Audiosignal vorgenommen werden.

In diesem Zusammenhang meint Korrigieren insbesondere, dass Frequenzen, die aufgrund eines nichtlinearen Frequenzgangs des Abspielgeräts gedämpft werden, im Audiosignal entsprechend erhöht und Frequenzen, die aufgrund des Abspielgeräts erhöht werden, im Audiosignal entsprechend gedämpft werden.

Ein Abspielgerät ist insbesondere ein Lautsprecher, ein Kopfhörer oder ein portables oder nichtportables Wiedergabegerät, beispielsweise eine Stereoanlage oder ein mp3-Player.

Das Korrigieren des ersten Audiosignals bzw. des zweiten Audiosignals erfolgt bevorzugt mittels eines Filters. Die hierfür benötigten Filterkoeffizienten entstammen beispielweise einer Datenbank, in der Filterkoeffizienten für verschiedene bekannte Abspielgeräte, beispielweise eine Vielzahl an erhältlichen Kopfhörermodellen, zur wiederholten Verwendung abgelegt oder gespeichert sind.

Ein bei einem erfindungsgemäßen Verfahren verwendeter Filter ist vorzugsweise ein Filter mit endlicher Impulsantwort. Derartige Filter werden auch als FIR-Filter (für Englisch: Finite Impulse Response, endliche Impulsantwort) oder Transversalfilter bezeichnet. Ein Filter mit endlicher Impulsantwort lässt sich vorteilhaft als digitaler Filter implementieren und ist konstruktionsbedingt stabil. Somit werden insbesondere ungewollte, durch den Filter angeregte Schwingungen ausgeschlossen.

Vorzugsweise wird der therapeutisch verwendbare Frequenzbereich in Frequenzintervalle unterteilt analysiert, wobei insbesondere jeweils eine auditive Energie des ersten Audiosignals oder des zweiten Audiosignals innerhalb eines jeden Frequenzintervalls ermittelt wird und der Bewertungsparameter in Abhängigkeit der jeweiligen auditiven Energie und eines jeweiligen Frequenzabstandes zwischen dem Sperrbereich und dem jeweiligen Frequenzintervall unter Berücksichtigung aller Frequenzintervalle bestimmt wird. Dadurch kann berücksichtigt werden, dass die laterale Hemmung mit steigendem Frequenzabstand in der Regel abnimmt, wodurch die Korrelation zwischen Bewertungsparameter und tatsächlicher Hemmung neuronaler Aktivität erhöht wird. Gleichzeitig wird durch die Verwendung von Frequenzintervallen der Aufwand für die Analyse gegenüber einer Analyse des kontinuierlichen Frequenzspektrums erheblich verringert.

Die Frequenzintervalle sind dabei vorzugsweise in Anlehnung an das menschliche Gehör gewählt und weisen beispielsweise jeweils eine Frequenzbreite von 1/3 bark oder 1/3 ERB auf. Diese beiden Skalen sind jeweils nicht-linear mit der Frequenz verknüpft und berücksichtigen das über weite Bereiche logarithmische Frequenzverhalten des menschlichen Gehörs.

Des Weiteren wird vorzugsweise zum Bestimmen des Bewertungsparameters das erste Audiosignal bzw. das zweite Audiosignal in zeitlich aufeinanderfolgende Abschnitte unterteilt analysiert, wobei insbesondere jeder Abschnitt eine vorgebbare Dauer oder eine vorgebbare Anzahl digitaler Audiosamples umfasst. Aufeinanderfolgende Abschnitte können im Rahmen der Erfindung beabstandete, überlappende oder aneinandergrenzende Abschnitte sein. Hierdurch wird erreicht, dass der Bewertungsparameter zeitabhängig mit einer zeitlichen Auflösung, die insbesondere von der Dauer eines Abschnitts abhängt, bestimmt wird. Bei einer Samplingrate oder Abtastrate von 44,1 kHz umfasst ein Abschnitt beispielsweise 576 Audiosamples, wodurch ein guter Kompromiss zwischen Frequenzauflösung und Zeitauflösung erreicht wird. Die Länge der Abschnitte kann auch variabel ausgebildet sein, um sowohl Abschnitte mit einer hohen Zeitauflösung und als auch Abschnitte mit einer hohen Frequenzauflösung zu erhalten.

Unter einem Audiosample wird insbesondere die Pulshöheninformation eines digitalisierten Audiosignals zu einem Zeitpunkt verstanden. Die Samplingrate oder Abtastrate gibt dabei insbesondere die zeitliche Digitalisierung oder Diskretisierung des Audiosignals an, d.h. wie viele Audiosamples pro Zeiteinheit in dem digitalen Audiosignal kodiert sind.

Wenn das erste Audiosignal bzw. das zweite Audiosignal wenigstens zwei Kanäle aufweist, wird zum Bestimmen des Bewertungsparameters vorzugsweise jeder Kanal einzeln analysiert. Dadurch können bei der Bestimmung oder Berechnung des Bewertungsparameters Phasenverschiebungen zwischen den beiden Kanälen und die entsprechenden Auswirkungen auf die laterale Hemmung der Neuronen in der Hörrinde berücksichtigt werden.

Alternativ kann der Bewertungsparameter anhand eines einzelnen Kanals oder aus einem Mischsignal mehrerer Kanäle bestimmt werden. Dies ist insbesondere vorteilhaft, wenn der Analyseaufwand zeitkritisch ist, beispielsweise bei Echtzeitanwendungen des erfindungsgemäßen Verfahrens.

Besonders bevorzugt wird ein erfindungsgemäßes Verfahren unter Verwendung einer Datenverarbeitungsvorrichtung ausgeführt.

Die Datenverarbeitungsvorrichtung ist insbesondere ausgebildet zur digitalen Bearbeitung analoger und/oder digitaler Audiosignale, wobei analoge Audiosignale beispielsweise mittels der Datenverarbeitungsvorrichtung vor Ausführen des erfindungsgemäßen Verfahrens digitalisiert werden.

Eine geeignete Datenverarbeitungsvorrichtung ist beispielsweise ein Server, ein Multimedia-Computer oder ein Laptop, die den Vorteil haben, dass sie für jedermann verfügbar sind.

Vorzugsweise ist oder wird die Datenverarbeitungsvorrichtung mittels einer ersten Datenverbindung mit einer Wiedergabevorrichtung verbunden, wobei das zweite Audiosignal von der Datenverarbeitungsvorrichtung über die erste Datenverbindung an die Wiedergabevorrichtung übertragen wird.

Eine Wiedergabevorrichtung ist insbesondere zum Wiedergeben von Audiosignalen ausgebildet und ist beispielsweise ein Computer oder Laptop, ein Smartphone oder ein mobiles Abspielgerät.

Geeignete Datenverbindungen werden beispielsweise über ein Netzwerk, wie Ethernet, LAN (für Englisch: Local Area Network) oder WLAN (für Englisch: Wireless Local Area Network), über Standardschnittstellen wie Bluetooth, USB (für Englisch: Universal Serial Bus) oder Infrarot oder als Telekommunikationsverbindung wie ISDN, DSL, GSM oder UMTS, bereitgestellt.

Vorzugsweise ist oder wird die Datenverarbeitungsvorrichtung mittels einer zweiten Datenverbindung mit einem Datenspeicher verbunden, wobei das erste Audiosignal vom Datenspeicher über die zweite Datenverbindung an die Datenverarbeitungsvorrichtung übertragen wird.

Ausdrücklich sind auch solche Ausführungsformen von der Erfindung umfasst, bei denen die Wiedergabevorrichtung als Datenspeieher ausgebildet ist. In diesem Fall können die erste Datenverbindung und die zweite Datenverbindung insbesondere identisch sein oder zeitlich nacheinander hergestellt werden.

Die erste Datenverbindung und/oder die zweite Datenverbindung ist oder wird bzw. sind oder werden vorzugsweise über ein Datennetzwerk, insbesondere über das Internet, hergestellt.

Unabhängig vom Format des ersten Audiosignals ist das zweite Audiosignal beispielsweise eine Audiodatei, die zum Abruf oder Download über das Datennetzwerk bereitgehalten wird. Das zweite Audiosignal kann auch ein Audiodatenstrom sein, der in Echtzeit über das Datennetzwerk an die Wiedergabevorrichtung übertragen wird.

Die der Erfindung zugrunde liegende Aufgabe wird des Weiteren gelöst durch ein Computerprogrammprodukt mit Programmcodemitteln, die dazu ausgebildet sind, ein erfindungsgemäßes Verfahren auszuführen, wenn die Programmcodemittel auf einer Datenverarbeitungsvorrichtung ausgeführt werden.

Vorzugsweise sind die Programmcodemittel auf einem von einem Computer lesbaren Datenträger gespeichert. Hierbei kann es sich um eine CD, eine Diskette, eine Festplatte oder auch einen Speicherplatz auf einem Server handeln. Die Speicherung kann zudem sowohl in einem RAM oder ROM oder einem zu einem Server ausgelagerten Festkörperspeicher oder Festplattenspeicher vorgesehen sein.

Im Rahmen der Erfindung werden insbesondere auch Programmcodemittel, die auf einem Internetserver gespeichert sind und als Download zur temporären oder permanenten Speicherung, Installation und/oder Verwendung auf einem Computer oder Laptop angeboten werden, als Computerprogrammprodukt verstanden.

Die der Erfindung zugrundeliegende Aufgabe wird ferner gelöst durch ein Computersystem mit einer Datenverarbeitungsvorrichtung, die dazu eingerichtet ist, ein erfindungsgemäßes Verfahren auszuführen.

Hierfür umfasst das Computersystem beispielsweise Programmcodemittel, die dazu ausgebildet sind, ein erfindungsgemäßes Verfahren auszuführen, wenn die Programmcodemittel auf der Datenverarbeitungsvorrichtung ausgeführt werden. Alternativ oder ergänzend verfügt die Datenverarbeitungsvorrichtung über geeignete Komponenten, beispielsweise elektronische Schaltkreise oder mikroelektronische Bauteile, zum Ausführen einzelner oder aller Verfahrensschritte.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: schematisch eine beispielhafte Implementierung des erfindungsgemäßen Verfahrens,
- Fig. 2: schematisch den Amplituden-Frequenzgang eines erfindungsgemäß verwendeten Filters,
- Fig. 3: schematisch ein Ablaufdiagramm eines Filters eines erfindungsgemäßen Verfahrens,
- Fig. 4: schematisch ein Ablaufdiagramm einer Signalanalyse eines erfindungsgemäßen Verfahrens,
- Fig. 5: schematisch eine weitere beispielhafte Implementierung des erfindungsgemäßen Verfahrens,
- Fig. 6: schematisch noch eine beispielhafte Implementierung des erfindungsgemäßen Verfahrens,
- Fig. 7a: schematisch einen Ausschnitt eines Ablaufdiagramms eines Filters eines erfindungsgemäßen Verfahrens und
- Fig. 7b: schematisch einen Ausschnitt eines Ablaufdiagramms einer Signalanalyse eines erfindungsgemäßen Verfahrens.

In den Zeichnungen sind jeweils gleiche oder gleichartige Elemente und/oder Teile mit denselben Bezugsziffern versehen, so dass von einer erneuten Vorstellung jeweils abgesehen wird.

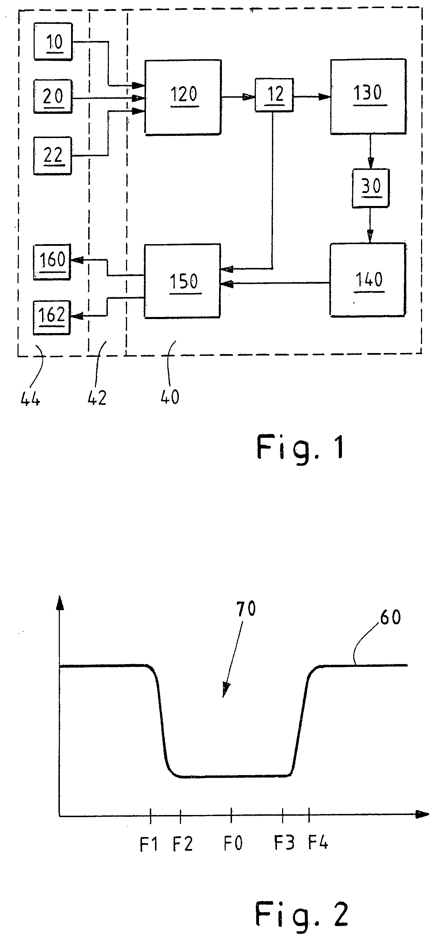

Eine beispielhafte Implementierung des erfindungsgemäßen Verfahrens ist in Fig. 1 dargestellt. Dabei ist ein Server 40 vorgesehen, der beispielsweise über ein entsprechendes Web-Front-End über das Internet 42 von einem Client-Rechner 44 zugänglich ist.

Über den Client-Rechner 44 wird ein ursprüngliches Audiosignal 10, beispielsweise eine auf dem Client-Rechner 44 gespeicherte Audiodatei, über das Internet 42 an den Server 40 übertragen, wo mittels eines digitalen Filters 120 ein Therapiesignal 12 erstellt wird. Zur Konfiguration des Filters werden über den Client-Rechner 44 eine individuelle Tinnitusfrequenz 20 oder Therapiefrequenz 20 und optional eine Sperrbreite 22, insbesondere Sperrbandbreite, bereitgestellt, die beispielsweise von dem behandelnden Arzt für den individuellen Tinnituspatienten bestimmt worden sind. Wenn keine Sperrbreite 22 angegeben wird, wird für die Sperrbreite 22 ein Standardwert, beispielweise eine Oktave, verwendet.

In einer Signalanalyse 130 wird das Therapiesignal 12 analysiert und wenigstens ein Bewertungsparameter 30 bestimmt. In Anlehnung an den Aussagegehalt, ein Maß für die Hemmung neuronaler Aktivität zu sein, wird der Bewertungsparameter 30 im Folgenden auch als Hemmungsparameter 30 bezeichnet.

In einer Parameterauswertung 140 wird der Hemmungsparameter 30 mit Referenzparametern verglichen, um die Eignung des Therapiesignals 12 für die Therapie oder Behandlung des individuellen Tinnitus mit der Tinnitustfrequenz 20 zu bestimmen.

Die Referenzparameter beruhen beispielsweise auf Referenzsignalen, die sich in empirischen Studien als geeignet oder ungeeignet für die Tinnitustherapie erwiesen haben, wobei die Referenzparameter durch die mittels der Signalanalyse 130 bestimmten Hemmungsparameter 30 für die Referenzsignale vorgegeben werden.

Das Ergebnis der Parameterauswertung 140 wird über einen Benutzerdialog 150 an den Client-Rechner 44 übermittelt. Gleichzeitig stellt der Benutzerdialog 150 einen Audiodatenstrom 160 mit dem Therapiesignal 12 zur Wiedergabe mittels des Client-Rechners 44 oder eine Audiodatei 162 mit dem Therapiesignal 12 zur Speicherung auf dem Client-Rechner 44 bereit.

Der Amplituden-Frequenzgang des Filters 120 ist in Fig. 2 schematisch in Form einer Kennlinie 60 dargestellt. Dabei entsprechen die horizontale Achse der Frequenz des zu filternden Audiosignals und die vertikale Achse der Dämpfung des Filters.

Die Kennlinie 60 des Filters 120 weist eine Bandsperre 70 um eine Mittenfrequenz F0, die insbesondere der individuellen Tinnitusfrequenz 20 entspricht, auf. Die Bandsperre 70 weist einen therapeutischen Zielbereich oder Sperrbereich mit einer Sperrbreite 22 auf, die beispielsweise eine Oktave beträgt oder als variable Sperrbreite 22 vorgegeben ist. Der Sperrbereich definiert eine untere Grenzfrequenz F2 des therapeutischen Zielbereichs bzw. Sperrbereichs und eine obere Grenzfrequenz F3 des therapeutischen Zielbereichs bzw. Sperrbereichs, wobei der Sperrbereich beispielsweise auf einer logarithmischen Frequenzskala symmetrisch um die Mittenfrequenz F0 angeordnet ist. Die Dämpfung der Bandsperre 70, insbesondere die Dämpfung des Filters im therapeutischen Zielbereich, wird insbesondere in Abhängigkeit der Quantisierungswortbreite M eines zu filternden digitalen Audiosignals 10 bestimmt und beträgt beispielsweise M * 6 dB + 2 dB.

Oberhalb und unterhalb des Sperrbereichs weist die Bandsperre 70 Übergangsbereiche auf, die durch die untere Grenzfrequenz F1 der Bandsperre 70 und die obere Grenzfrequenz F4 der Bandsperre 70 charakterisiert sind. Die Breite der Übergangsbereiche ist dabei abhängig von der Implementierung des jeweiligen Filters 120, wobei in der Regel eine abnehmende Breite der Übergangsbereiche bei einem digital implementierten Filter 120 mit erhöhtem Rechenaufwand und somit mit erhöhtem Zeitaufwand bei dem Erstellen des Therapiesignals 12 verbunden ist. Vorzugsweise ist der Filter 120 derart ausgebildet oder konfiguriert, dass die Breite der Übergangsbereiche klein ist gegenüber der Sperrbreite 22. Beispielsweise beträgt die Breite der Übergangsbereiche jeweils einen Viertelton, wenn die Sperrbreite 22 eine Oktave bzw. sechs Ganztonschritte beträgt.

Außerhalb der Übergangsbereiche weist die Kennlinie 60 des Filters 120 jeweils Durchlassbereiche auf, in denen das zu filternde Audiosignal im Wesentlichen unverändert erhalten bleibt. In diesen Bereichen ist die Dämpfung entsprechend null.

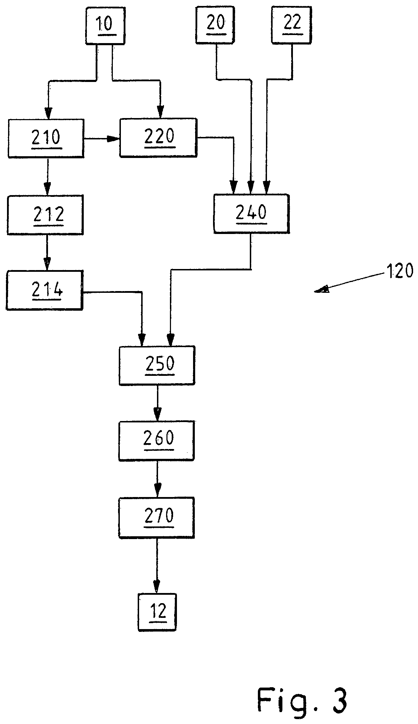

In Fig. 3 ist eine beispielhafte Implementierung des digitalen Filters 120 dargestellt. Eingangsparameter für das Filter 120 sind das ursprüngliche Audiosignal 10, das insbesondere ein digitales Audiosignal ist, die individuelle Tinnitusfrequenz 20 sowie die Sperrbreite 22.

In einer Signalaufbereitung 210 wird das ursprüngliche Audiosignal 10 dekodiert und ggf. in ein lineares PCM-Format (für Englisch: Pulse Code Modulation, Pulshöhenmodulation) überführt, sofern das ursprüngliche Audiosignal 10 noch nicht in einem solchen Format vorliegt.

Das derart aufbereitete Audiosignal wird einer Normalisierung 212 unterzogen, um den Signal-Rauschabstand des gefilterten Audiosignals gering zu halten. Falls die Sprungantwort des Filters 120 Überschwinger produziert, wird zudem in einer linearen Dämpfung 214 das Audiosignal etwa um die Höhe der Überschwinger reduziert, um Verzerrungen im gefilterten Audiosignal zu vermeiden.

Des Weiteren werden in einer Parameterbestimmung 220 die Abtastrate des Audiosignals 10 sowie die Quantisierungswortbreite M des aufbereiteten Audiosignals bestimmt.

Die eigentliche Filterung des Audiosignals erfolgt mittels eines FIR-Filters 250 durch numerische Faltung mit geeigneten Filterkoeffizienten, die zuvor unter Berücksichtigung der Abtastrate, der Quantisierungswortbreite M, der individuellen Tinnitusfrequenz 20 sowie der Sperrbreite 22 bestimmt wurden (Block 240).

In einer anschließenden Rauschunterdrückung 260, dem so genannten "Dithering", werden Digitalisierungsrundungen im gefilterten Audiosignal randomisiert. Abschließend wird in einer Signalnachbereitung 270 das gefilterte Audiosignal in ein frei wählbares Datenformat konvertiert und als Therapiesignal 12 bereitgestellt. Beispielsweise wird das Datenformat des ursprünglichen Audiosignals 10 verwendet.

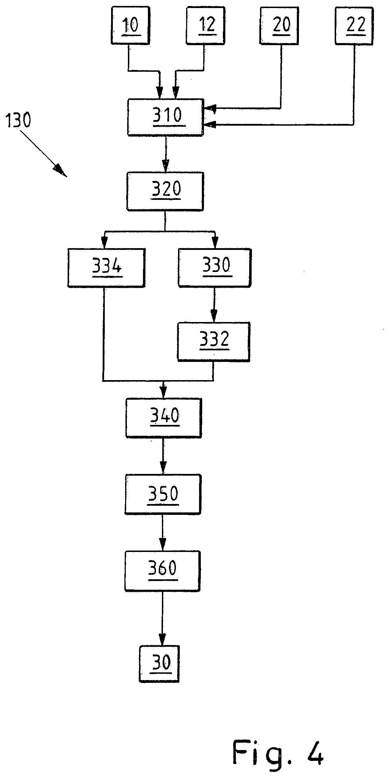

Fig. 4 zeigt schematisch ein Ablaufdiagramm einer beispielhaften Implementierung der Signalanalyse 130. Eingangsseitig werden der Signalanalyse 130 das ursprüngliche Audiosignal 10 oder das Therapiesignal 12, die Tinnitusfrequenz 20 sowie die Sperrbreite 22 zugeführt.

Das zu analysierende Audiosignal 10, 12 wird abschnittsweise analysiert, wobei ein Abschnitt beispielsweise 576 Audiosamples bei einer Abtastrate von 44,1 kHz umfasst und im Folgenden als Granulum bezeichnet wird. Zudem können, sofern vorhanden, der linke Stereokanal und der rechte Stereokanal des Audiosignals einzeln analysiert werden.

Jedes Granulum des zu analysierenden Audiosignals 10, 12 wird im Frequenzbereich in Anlehnung an die Funktionsweise des menschlichen Gehörs analysiert. Der Modellbildung des menschlichen Gehörs werden in der Regel auditive Filter mit unterschiedlicher und meist relativer Bandbreite zugrunde gelegt. Solche sind beispielsweise die Frequenzgruppen nach Zwicker, d.h. die sogenannte bark-Skala, oder die äquivalente Rechteckbandbreite, d.h. die sogenannte ERB-Skala (für Englisch: Equivalent Rectangular Bandwidth) nach Moore. Sowohl die bark-Skala als auch die ERB-Skala sind nicht-linear mit der Frequenz verknüpft und so gewählt, dass die Unterteilung der Skala in ganzzahlige Skalenabschnitte der Signalverarbeitung des menschlichen Gehörs entspricht. Für eine differenziertere Analyse kann jeder Skalenabschnitt jeweils in mehrere, beispielsweise drei, Teile geteilt werden. Ein solcher Teil wird im Folgenden als Partitionsband bezeichnet und hat beispielsweise eine Breite von 1/3 bark oder 1/3 ERB.

In jedem Granulum des zu analysierenden Audiosignals 10, 12 wird für jedes Partitionsband eine in dem Partitionsband enthaltene auditive Energie bestimmt (Block 310). Dies erfolgt beispielsweise unter Verwendung einer schnellen Fourier-Transformation oder Fast Fourier Transformation, FFT, und unter Annahme eines Schalldruckpegels, der beim Abhören des Audiosignals 10, 12 zu einer als moderat empfundenen Lautstärke führt. Beispielsweise wird das zu analysierende Audiosignal 10, 12 dabei derart skaliert, dass der maximale Schalldruckpegel etwa 70 dB beträgt.

Des Weiteren wird in jedem Granulum eine Tonalität für jedes Partitionsband bestimmt (Block 320). Die Tonalität ist ein Maß dafür, ob ein Schallereignis geräuschartig, d.h. breitbandig, oder tonal, d.h. schmalbandig, ist. Sie kann beispielsweise über die Vorhersagbarkeit oder Periodizität des Audiosignals über die Zeit bestimmt werden, wobei hierfür eine Betrachtung mehrerer aufeinanderfolgender zeitlicher Abschnitte des zu analysierenden Audiosignals 10, 12 erforderlich ist. Bevorzugt sind daher alternative Bestimmungsverfahren, beispielsweise anhand der Verteilung der Schallenergie im Frequenzspektrum des aktuell analysierten Granulums, insbesondere innerhalb der einzelnen Partitionsbänder des Granulums.

Wenn das aktuelle Partitionsband ganz oder teilweise außerhalb des therapeutischen Zielbereichs liegt, so wird zunächst anhand der auditiven Energie und der Tonalität eine Erregungsstärke des aktuellen Partitionsbandes eine Erregungsstärke bestimmt (Block 330), wobei berücksichtigt werden kann, dass geräuschartige Schallereignisse bei gleichem Schalldruck stärker oder lauter wahrgenommen werden als tonale Schallereignisse. Ebenso kann berücksichtigt werden, dass höhere Frequenzen bei gleicher Schallenergie schwächer oder weniger laut wahrgenommen werden als tiefere Töne, indem beispielsweise die Erregungsstärke reduziert wird, wenn das aktuelle Partitionsband oberhalb der Tinnitusfrequenz 20 bzw. oberhalb des therapeutischen Zielbereichs liegt. Die Erregungsstärke ist ein Maß für die Stimulierung der zu dem aktuellen Partitionsband tonotopen Neuronen der primären Hörrinde.

Aus der Erregungsstärke für das aktuelle Partitionsband wird für alle anderen Partitionsbänder eine Dämpfungsstärke bestimmt (Block 332), die ein Maß für die laterale Hemmung der zu den anderen Partitionsbändern jeweils tonotopen Neuronen ist. Hierfür werden insbesondere neuroakustische oder psychoakustische Spreadingfunktionen verwendet. Dabei wird insbesondere berücksichtigt, dass die Reichweite der lateralen Hemmung stark abhängig ist von der Erregungsstärke oder der Stärke der Stimulierung der zum aktuellen Partitionsband tonotopen Neuronen. Je größer die Erregungsstärke, desto größer ist der Frequenzbereich bzw. die Anzahl benachbarter Partitionsbänder, in denen die laterale Hemmung relevante Auswirkungen zeigt. Kommen empirisch bestimmte psychoakustische Spreadingfunktionen zum Einsatz, so erfolgt vorzugsweise eine Korrektur anhand der Frequenzkurven gleicher Lautstärke (Isophone) nach ISO 226:2003, um insbesondere frequenzbewertende Eigenschaften des Außen-, Mittel- und Innenohres zu kompensieren.

Sofern das aktuelle Partitionsband innerhalb des therapeutischen Zielbereichs, der insbesondere durch die Tinnitusfrequenz 20 und die Sperrbreite 22 vorgegeben ist, liegt, wird ebenfalls eine Erregungsstärke bestimmt (Block 334). Dabei wird danach unterschieden, ob das zu analysierende Audiosignal 10, 12 ein ungefiltertes oder ursprüngliches Audiosignal 10 oder ein Therapiesignal 12 ist.

Im Falle eines ungefilterten Audiosignals 10 wird die Erregungsstärke auf null gesetzt. Das ungefilterte Audiosignal 10 wird entsprechend so behandelt, als wenn es mit einem ideal dämpfenden Bandsperrfilter mit unendlich schmalen Übergangsbereichen bearbeitet worden wäre.

Im Falle eines gefilterten Audiosignals oder eines Therapiesignals 12 wird die Erregungsstärke wie im Falle eines Partitionsbandes außerhalb des therapeutischen Zielbereichs bzw. Sperrbereichs bestimmt.

Die vorbeschriebenen Analyseschritte 310, 320, 330, 332, 334 werden für alle Partitionsbänder eines Granulums wiederholt. Danach stehen für jedes Partitionsband des Granulums eine Erregungsstärke sowie eine Vielzahl von Dämpfungsstärken zur Verfügung.

Die Dämpfungsstärken für jedes Partitionsband werden jeweils zu einer Gesamtdämpfungsstärke für dieses Partitionsband zusammengefasst (Block 340). Dies erfolgt beispielsweise mittels Intensitätsaddition, mittels nichtlinearer Addition oder mittels Maximalwertberechnung.

Optional werden die Erregungsstärken sowie die Gesamtdämpfungsstärken aller Partitionsbänder eines Granulums bezüglich solcher eines oder mehrerer anderer Granula korrigiert (Block 350).

Durch eine Korrektur bezüglich eines oder mehrerer vorangegangener Granula kann beispielsweise berücksichtigt werden, dass eine starke Erregung oder Dämpfung eines neuronalen Areals auch nach Abklingen des Stimulus für kurze Zeit nachwirkt.

Entsprechend kann durch Korrektur bezüglich eines zeitgleichen Granulums für einen anderen Kanal des Audiosignals 10, 12 berücksichtigt werden, dass gegebenenfalls eine Erregung der für ein Ohr zuständigen Neuronen eine Dämpfung der für das andere Ohr zuständigen Neuronen zur Folge hat.

Die Gesamtdämpfungsstärken derjenigen Partitionsbänder, die innerhalb des therapeutischen Zielbereichs liegen, werden abschließend zu einem Hemmungsparameter 30 zusammengefasst (Block 360), was beispielsweise mittels Intensitätsaddition, mittels nicht-linearer Addition oder mittels Maximalwertberechnung erfolgt. Sofern das zu analysierende Audiosignal ein Therapiesignal 12 ist, werden dabei die Erregungsstärken der innerhalb des Sperrbereichs liegenden Partialbänder mit entgegengesetztem Vorzeichen ebenfalls einbezogen.

Für jedes Granulum steht somit abschließend ein Hemmungsparameter 30 zur Verfügung, der ein Maß für eine Hemmung neuronaler Aktivität in der primären Hörrinde beim Hören des aktuellen Granulums des analysierten Therapiesignals 12 bzw. eines aus dem analysierten ungefilterten Audiosignal 10 erstellten Therapiesignals ist.

Aus den Hemmungsparametern 30 können weitere Parameter ermittelt werden, die ebenfalls mit der Hemmung neuronaler Aktivität in der primären Hörrinde korrelieren.

Insbesondere können die zeitgleichen Granuli zweier Stereokanäle zu einem Summenparameter und zu einem Differenzparameter zusammengefasst werden. Der Summenparameter, für den die Hemmungsparameter 30 der Granuli beider Stereokanäle insbesondere mit gleichem Vorzeichen berücksichtigt werden, ist beispielswiese ein Maß für das Therapiepotenzial des Audiosignals 10, 12. Dies gilt auch für den Fall, dass die Therapie mittels Lautsprecher erfolgt und somit beide Ohren beiden Stereokanälen gleichermaßen ausgesetzt sind. Der Differenzparameter, für den die Hemmungsparameter 30 der Granuli beider Stereokanäle insbesondere mit unterschiedlichem Vorzeichen berücksichtigt werden, gibt hingegen beispielsweise an, wie sich das Therapiepotenzial des Audiosignals 10, 12 auf die Stereokanäle verteilt. Dies ist insbesondere interessant für den Fall, dass die Therapie mittels Kopfhörer erfolgt und somit jeweils ein Ohr einem Stereokanal ausgesetzt ist.

Es können auch die Hemmungsparameter 30, die Summenparameter oder die Differenzparameter aller Granuli eines Audiosignals 10, 12 zu einem Gesamtparameter zusammengefasst werden, der entsprechend insbesondere das Therapiepotenzial des gesamten Audiosignals 10, 12 angibt. Dies erfolgt jeweils beispielsweise mittels Intensitätsaddition, nicht-linearer Addition, Maximalwertbildung oder auch Mittelwertbildung.

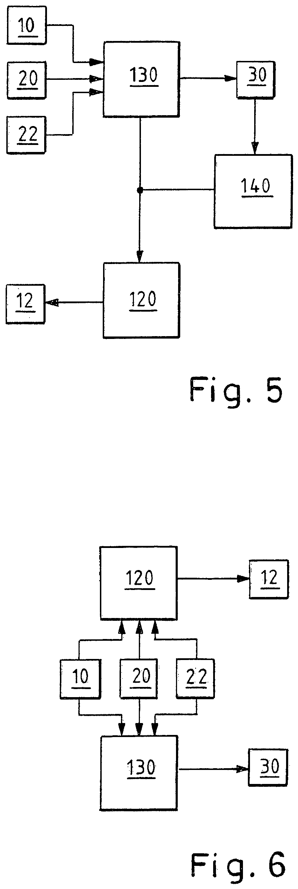

Fig. 5 zeigt schematisch eine weitere Implementierung des erfindungsgemäßen Verfahrens, die sich von der Implementierung gemäß Fig. 1 dadurch unterscheidet, dass zuerst die Signalanalyse 130 ausgeführt und der Hemmungsparameter 30 bestimmt wird.

Dieser wird dann in der Parameterauswertung 140 ausgewertet, wobei das Therapiesignal 12 mittels des Filters 120 nur erfolgt, wenn die Parameterauswertung 140 eine ausreichende Therapieeignung des ursprünglichen Audiosignals 10 ergeben hat.

Die Implementierungen gemäß Fig. 1 und Fig. 5 können auch kombiniert werden, wobei dann die Signalanalyse 130 sowohl auf dem ursprünglichen Audiosignal 10 als auch auf dem Therapiesignal 12 ausgeführt wird.

Fig. 6 zeigt eine weitere Ausführungsform des erfindungsgemäßen Verfahrens, die sich insbesondere für Echtzeitanwendungen eignet. Hierbei werden der Filter 120 und die Signalanalyse 130 parallel ausgeführt, so dass das Therapiesignal 12 und der Hemmungsparameter 30 gleichzeitig zur Verfügung stehen.

Das erfindungsgemäße Verfahren ist auch geeignet zur Bereitstellung oder Bearbeitung von Audiosignalen für eine Tinnitustherapie zur Verwendung mit Abspielgeräten, die einen nichtlinearen Frequenzgang aufweisen.

Beispielsweise weisen handelsübliche Kopfhörer oftmals bauartbedingt oder gezielt manipuliert einen nichtlinearen Frequenzgang auf, wobei die Nichtlinearität in der Regel für alle Modelle einer Serie homogen und entsprechend bekannt oder zumindest ermittelbar ist.

Durch die Verwendung eines nichtlinearen Wiedergabegerätes oder eines Wiedergabegerätes mit nichtlinearem Frequenzgang werden die therapeutischen Qualitäten des für die Tinnitustherapie bereitgestellten Audiosignals gemindert und die Bewertung der Therapieeignung des Audiosignals gemäß vorstehender Beschreibung verfälscht.

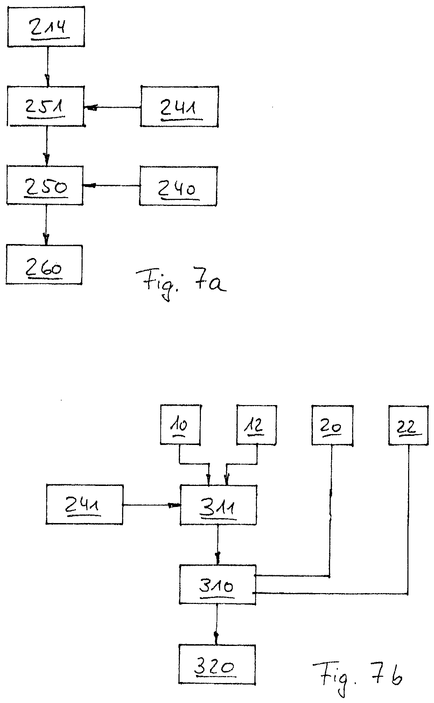

Um dies zu verhindern, ist im Rahmen der Erfindung eine optionale Korrektur des für die Therapie bereitgestellten Audiosignals vorgesehen. Eine beispielhafte Ausführung dieser Korrektur ist in den Fig. 7a und 7b beschrieben.

Fig. 7a zeigt schematisch einen Ausschnitt eines Ablaufdiagramms eines Filters 120 für ein erfindungsgemäßes Verfahren. Das Filter 120 entspricht dem in Fig. 3 dargestellten Filter 120, wobei der in Fig. 7a gezeigte Ausschnitt die Blöcke 214, 240 und 250 in Fig. 3 ersetzt.

Vor dem FIR-Filter 250 wird ein weiteres, beispielsweise als FIR-Filter ausgebildetes, Korrekturfilter 251 angewendet, mittels dessen eine Korrektur bezüglich der Nichtlinearität des Abspielgerätes vorgenommen wird. Hierfür werden beispielsweise Filterkoeffizienten 241 oder Korrekturkoeffizienten 241 aus einer Datenbank verwendet, die an das zu korrigierende Wiedergabegerät angepasst sind. Durch das Korrekturfilter 251 werden solche Frequenzen, die aufgrund der Nichtlinearität des Wiedergabegerätes gedämpft wiedergegeben werden, in dem gefilterten Audiosignal erhöht. Entsprechend werden in dem gefilterten Audiosignal solche Frequenzen gedämpft, die aufgrund der Nichtlinearität des Wiedergabegeräts überhöht oder verstärkt wiedergegeben werden.

Vorzugsweise wird die erfolgte Korrektur in dem zur Therapie bereitgestellten Audiosignal 12 ebenfalls bei der Bestimmung des Hemmungsparameters 30 berücksichtigt, wie in Fig. 7b dargestellt ist. Fig. 7b zeigt einen Ausschnitt eines Ablaufdiagramms vergleichbar mit Fig. 4, wobei beispielsweise der obere Teil der Darstellung in Fig. 4 durch den Ausschnitt in Fig. 7b ersetzt wird.

Hier wird vor der Bestimmung der auditiven Energie (Block 310) eine Nichtlinearitätssimulation 311 vorgenommen, um die Nichtlinearität des Wiedergabegeräts korrekt zu berücksichtigen. Die Nichtlinearitätssimulation 311 basiert dabei auf den bereits für das Korrekturfilter 251 verwendeten Korrekturkoeffizienten 241.

### Bezugszeichenliste

- 10: ungefiltertes Audiosignal
- 12: Therapiesignal
- 20: Tinnitusfrequenz
- 22: Sperrbreite
- 30: Hemmungsparameter
- 40: Server
- 42: Internet
- 44: Client-Rechner
- 60: Kennlinie des Filters
- 70: Bandsperre
- 120: Filter
- 130: Signalanalyse
- 140: Parameterauswertung
- 150: Benutzerdialog
- 160: Audiodatenstrom
- 162: Audiodatei
- 210: Signalaufbereitung
- 212: Normalisierung
- 214: Dämpfung
- 220: Parameterbestimmung
- 240: Koeffizientenbestimmung
- 241: Korrekturkoeffizienten
- 250: FIR-Filter
- 251: Korrekturfilter
- 260: Rauschunterdrückung
- 270: Signalnachbereitung
- 310: Energiebestimmung
- 311: Nichtlinearitätssimulation
- 320: Tonalitätsbestimmung
- 330: Erregungsstärkenbestimmung
- 332: Dämpfungsstärkenbestimmung
- 334: Erregungsstärkenbestimmung
- 340: Kombination
- 350: Korrektur
- 360: Hemmungsparameterbestimmung
- F0: Mittenfrequenz
- F1: Untere Grenzfrequenz Bandsperre
- F2: Untere Grenzfrequenz therapeutischer Zielbereich
- F3: Obere Grenzfrequenz therapeutischer Zielbereich
- F4: Obere Grenzfrequenz Bandsperre
- M: Quantisierungswortbreite

## Patentansprüche

1. Verfahren zum Bearbeiten von Audiosignalen (10, 12), insbesondere für eine Therapie eines subjektiven Tinnitus mit einer individuellen Tinnitusfrequenz, umfassend die folgenden Verfahrensschritte:
- Bereitstellen eines ersten Audiosignals (10),
- Bestimmen eines Sperrbereichs im Frequenzspektrum des ersten Audiosignals (10) mit einer vorgebbaren Frequenzbreite (22) anhand einer vorgebbaren Therapiefrequenz (20),
- Erstellen eines zweiten Audiosignals (12) aus dem ersten Audiosignal (10) unter Verwendung eines Filters (120, 121) für einen Signalanteil des ersten Audiosignals (10) im Sperrbereich, wobei der Filter (120, 121) ein Bandsperrfilter ist, durch den Signalanteile des ersten Audiosignals (10) mit Frequenzen im Sperrbereich beim Erstellen des zweiten Audiosignals (12) ganz oder teilweise entfernt werden,
- Ermitteln einer auditiven Energie des ersten Audiosignals (10) oder des zweiten Audiosignals (12) innerhalb wenigstens eines vorgegebenen oder vorgebbaren therapeutisch verwendbaren Frequenzbereichs, und
- Bestimmen eines Bewertungsparameters (30) für das zweite Audiosignal (12) in Abhängigkeit von der auditiven Energie und von einem Frequenzabstand zwischen dem therapeutisch verwendbaren Frequenzbereich und dem Sperrbereich, wobei der Bewertungsparameter (30) geeignet definiert ist, um anzugeben, wie stark die Aktivität der zu der Therapiefrequenz (20) oder zum Sperrbereich tonotopen Neuronen durch laterale Hemmung aufgrund der Stimulierung von zum therapeutisch verwendbaren Frequenzbereich tonotopen Neuronen beim Hören des zweiten Audiosignals (12) gehemmt wird.

2. Verfahren nach Anspruch 1, **dadurch gekennzeichnet, dass** das erste Audiosignal (10) und/oder das zweite Audiosignal (12) jeweils ein digitales Audiosignal, insbesondere eine digitale Audiodatei oder ein digitaler Audiodatenstrom, ist.

3. Verfahren nach Anspruch 1 oder 2, **dadurch gekennzeichnet, dass** das erste Audiosignal (10) vor dem Erstellen des zweiten Audiosignals (12) normalisiert wird.

4. Verfahren nach einem der Ansprüche 1 bis 3, **dadurch gekennzeichnet, dass** das erste Audiosignal (10) und/oder das zweite Audiosignal (12) zum Ausgleich von frequenzabhängigen Überhöhungen und/oder Dämpfungen durch ein Abspielgerät mit nichtlinearem Frequenzgang korrigiert wird.

5. Verfahren nach Anspruch 4, **dadurch gekennzeichnet, dass** das Korrigieren des ersten Audiosignals (10) bzw. des zweiten Audiosignals (12) mittels eines Filters (251) erfolgt.

6. Verfahren nach einem der Ansprüche 1 bis 4, **dadurch gekennzeichnet, dass** der verwendete Filter (120, 121) ein Filter mit endlicher Impulsantwort ist.

7. Verfahren nach einem der Ansprüche 1 bis 6, **dadurch gekennzeichnet, dass** der therapeutisch verwendbare Frequenzbereich in Frequenzintervalle unterteilt analysiert wird, wobei insbesondere jeweils eine auditive Energie des ersten Audiosignals (10) oder des zweiten Audiosignals (12) innerhalb eines jeden Frequenzintervalls ermittelt wird und der Bewertungsparameter (30) in Abhängigkeit der jeweiligen auditiven Energie und eines jeweiligen Frequenzabstandes zwischen dem Sperrbereich und dem jeweiligen Frequenzintervall unter Berücksichtigung aller Frequenzintervalle des therapeutisch verwendbaren Frequenzbereichs bestimmt wird.

8. Verfahren nach einem der Ansprüche 1 bis 7, **dadurch gekennzeichnet, dass** zum Bestimmen des Bewertungsparameters (30) das erste Audiosignal (10) bzw. das zweite Audiosignal (12) in zeitlich aufeinanderfolgende Abschnitte unterteilt analysiert wird, wobei insbesondere jeder Abschnitt eine vorgebbare Dauer oder eine vorgebbare Anzahl digitaler Audiosamples umfasst.

9. Verfahren nach einem der Ansprüche 1 bis 8, **dadurch gekennzeichnet, dass** das erste Audiosignal (10) bzw. das zweite Audiosignal (12) wenigstens zwei Kanäle aufweist, wobei zum Bestimmen des Bewertungsparameters (30) jeder Kanal einzeln analysiert wird.

10. Verfahren nach einem der Ansprüche 1 bis 9, **dadurch gekennzeichnet, dass** das Verfahren unter Verwendung einer Datenverarbeitungsvorrichtung (40) ausgeführt wird.

11. Verfahren nach Anspruch 10, **dadurch gekennzeichnet, dass** die Datenverarbeitungsvorrichtung (40) mittels einer ersten Datenverbindung mit einer Wiedergabevorrichtung (44) verbunden ist oder wird, wobei das zweite Audiosignal (12) von der Datenverarbeitungsvorrichtung (40) über die erste Datenverbindung an die Wiedergabevorrichtung (44) übertragen wird.

12. Verfahren nach Anspruch 10 oder 11, **dadurch gekennzeichnet, dass** die Datenverarbeitungsvorrichtung (40) mittels einer zweiten Datenverbindung mit einem Datenspeicher (44) verbunden ist oder wird, wobei das erste Audiosignal (10) vom Datenspeicher (44) über die zweite Datenverbindung an die Datenverarbeitungsvorrichtung (40) übertragen wird.

13. Verfahren nach Anspruch 11 oder 12, **dadurch gekennzeichnet, dass** die erste Datenverbindung und/oder die zweite Datenverbindung über ein Datennetzwerk (42), insbesondere über das Internet, hergestellt ist oder wird bzw. sind oder werden.

14. Computerprogrammprodukt mit Programmcodemitteln, die dazu ausgebildet sind, ein Verfahren gemäß einem der Ansprüche 1 bis 13 auszuführen, wenn die Programmcodemittel auf einer Datenverarbeitungsvorrichtung (40) ausgeführt werden.

15. Computersystem mit einer Datenverarbeitungsvorrichtung (40), die dazu eingerichtet ist, ein Verfahren nach einem der Ansprüche 1 bis 13 auszuführen.

## Claims

1. A method for processing audio signals (10, 12), in particular for a therapy of subjective tinnitus with an individual tinnitus frequency, comprising the following method steps:
- provision of a first audio signal (10),
- determination of a blocking range in the frequency spectrum of the first audio signal (10) with a predefinable frequency width (22) on the basis of a predefinable therapy frequency (20),
- creation of a second audio signal (12) from the first audio signal (10) using a filter (120, 121) for a portion of the signal in the first audio signal (10) in the blocking range, wherein the filter (120, 121) is a band-stop filter, through which a portion of the signal of the first audio signal (10) with frequencies in the blocking range is completely or partially removed during creation of the second audio signal (10),
- determination of an auditory energy of the first audio signal (10) or of the second audio signal (12) within at least one predefined or predefinable therapeutically applicable frequency range and
- specification of an evaluation parameter (30) for the second audio signal (12) as a function of the auditory energy and of a frequency separation between the therapeutically applicable frequency range and the blocking range, whereas the evaluation parameter is suitably designed to specify how strong the activity of the tonotopic neutrons to the therapy frequency or respectively to the blocking range is inhibited by lateral inhibition based on the stimulation of tonotopic neurons to the therapeutically applicable frequency range when listening to the second audio signal.

2. The method according to claim 1 **characterized in that** the first audio signal (10) and/or the second audio signal (12) is respectively a digital audio signal, in particular a digital audio file or a digital audio data flow.

3. The method according to claim 1 or 2, **characterized in that** the first audio signal (10) is normalized before the creation of the second audio signal (12).

4. The method according to one claims 1 to 3, **characterized in that** the first audio signal (10) and/or the second audio signal (12) is corrected to compensate for frequency-dependent elevations and/or dampings by a playback device with a non-linear frequency path.

5. The method according to claim 4, **characterized in that** the correction of the first audio signal (10) or respectively of the second audio signal (12) is carried out by means of a filter (121, 120).

6. The method according to one of claims 1 to 4, **characterized in that** the used filter (120, 121) is a filter with finite impulse response.

7. The method according to one of claims 1 to 6, **characterized in that** the therapeutically applicable frequency range is analyzed subdivided into frequency intervals, wherein in particular respectively an auditory energy of the first audio signal (10) or of the second audio signal (12) is determined within each frequency interval and the evaluation parameter (30) is determined depending on the respective auditory energy and of a respective frequency distance between the blocking range and the respective frequency interval taking all frequency intervals of the therapeutically applicable frequency range into consideration.

8. The method according to one of claims 1 to 7, **characterized in that** the first audio signal (10) or respectively the second audio signal (12) is analyzed subdivided into temporally consecutive sections for determining the evaluation parameter (30), wherein in particular each section comprises a predefinable duration or a predefinable number of digital audio samples.

9. The method according to one of claims 1 to 8, **characterized in that** the first audio signal (10) or respectively the second audio signal (12) has at least two channels, wherein each channel is analyzed individually for determining the evaluation parameter (30).

10. The method according to one of claims 1 through 9, **characterized in that** the method is performed using a data processing device (40).

11. The method according to claim 10, **characterized in that** the data processing device (40) is or will be connected with a playback device (44) by means of a first data connection, wherein the second audio signal (12) is transmitted by the data processing device (40) to the playback device (44) via the first data connection.

12. The method according to claim 10 or 11, **characterized in that** the data processing device (40) is or will be connected with a data storage device (44) by means of a second data connection, wherein the first audio signal (10) is transmitted by the data storage device (44) to the data processing device (40) via the second data connection.

13. The method according to claim 11 or 12, **characterized in that** the first data connection and/or the second data connection is or will be or respectively are or will be established via a data network (42), in particular via the Internet.

14. A computer program product with program code means, the program code means being designed to execute a method according to one of claims 1 to 13 when the program code means are executed on a data processing device (40).

15. A computer system with a data processing device (40), which is set up to execute a method according to one of claims 1 to 13.

## Revendications

1. Procédé de traitement de signaux audio (10, 12), en particulier pour une thérapie d'acouphènes subjectifs ayant une fréquence d'acouphène individuelle, comprenant les étapes consistant à :
- générer un premier signal audio (10),
- déterminer une zone de blocage dans le spectre de fréquence du premier signal audio (10) ayant une étendue en fréquence (22) prédéfinissable au moyen d'une fréquence de thérapie (20) prédéfinissable,
- générer un deuxième signal audio (12) à partir du premier signal audio (10) en utilisant un filtre (120, 121) pour une partie de signal du premier signal audio (10) dans la zone de blocage, le filtre (120, 121) étant un filtre coupe-bande par lequel les parties de signal du premier signal audio (10) ayant des fréquences dans la zone de blocage sont totalement ou partiellement supprimées lorsque le deuxième signal audio (12) est généré,
- déterminer une énergie auditive du premier signal audio (10) ou du deuxième signal audio (12) dans au moins une gamme de fréquences thérapeutiquement utilisable prédéfinie ou prédéfinissable, et
- déterminer un paramètre d'évaluation (30) pour le deuxième signal audio (12) en fonction de l'énergie auditive et d'un écart de fréquence entre la gamme de fréquences thérapeutiquement utilisable et la zone de blocage, le paramètre d'évaluation (30) étant défini de manière appropriée pour indiquer dans quelle mesure l'activité des neurones tonotopiques à la fréquence de thérapie (20) ou à la zone de blocage est inhibée par une inhibition latérale due à la stimulation des neurones tonotopiques à la zone de fréquence thérapeutiquement utilisable lors de l'écoute du deuxième signal audio (12).

2. Procédé selon la revendication 1, **caractérisé en ce que** le premier signal audio (10) et/ou le deuxième signal audio (12) est respectivement un signal audio numérique, en particulier un fichier audio numérique ou un flux de données audio numérique.

3. Procédé selon la revendication 1 ou la revendication 2, **caractérisé en ce que** le premier signal audio (10) est normalisé avant que le deuxième signal audio (12) ne soit généré.

4. Procédé selon l'une des revendications 1 à 3, **caractérisé en ce que** le premier signal audio (10) et/ou le deuxième signal audio (12) est corrigé par un lecteur à réponse en fréquence non linéaire pour compenser les pics et/ou les atténuations dépendant de la fréquence.

5. Procédé selon la revendication 4, **caractérisé en ce que** la correction du premier signal audio (10) et/ou du deuxième signal audio (12) est effectuée au moyen d'un filtre (251).

6. Procédé selon l'une quelconque des revendications 1 à 4, **caractérisé en ce que** le filtre (120, 121) utilisé est un filtre à réponse impulsionnelle finie.

7. Procédé selon l'une des revendications 1 à 6, **caractérisé en ce que** la gamme de fréquences thérapeutiquement utilisable est analysée en étant divisée en intervalles de fréquence, une énergie auditive du premier signal audio (10) ou du deuxième signal audio (12) étant en particulier déterminée à l'intérieur de chaque intervalle de fréquence et le paramètre d'évaluation (30) en fonction de l'énergie auditive respective et d'un écart de fréquence respectif entre la zone de blocage et l'intervalle de fréquence respectif étant déterminé en tenant compte de tous les intervalles de fréquence de la gamme de fréquences thérapeutiquement utilisable.

8. Procédé selon l'une des revendications 1 à 7, **caractérisé en ce que**, pour déterminer le paramètre d'évaluation (30), le premier signal audio (10) ou le deuxième signal audio (12) est analysé en étant divisé en portions successives dans le temps, chaque portion comprenant notamment une durée prédéfinissable ou un nombre prédéfinissable d'échantillons audio numériques.

9. Procédé selon l'une des revendications 1 à 8, **caractérisé en ce que** le premier signal audio (10) ou le deuxième signal audio (12) comporte au moins deux canaux, chaque canal étant analysé individuellement pour déterminer le paramètre d'évaluation (30).

10. Procédé selon l'une des revendications 1 à 9, **caractérisé en ce que** le procédé est mis en oeuvre en utilisant un dispositif (40) de traitement de données.

11. Procédé selon la revendication 10, **caractérisé en ce que** le dispositif (40) de traitement de données est relié ou est apte à être relié à un appareil de lecture (44) au moyen d'une première connexion de données, le deuxième signal audio (12) étant transmis depuis le dispositif (40) de traitement de données à l'appareil de lecture (44) par l'intermédiaire de la première connexion de données.

12. Procédé selon la revendication 10 ou la revendication 11, **caractérisé en ce que** le dispositif (40) de traitement de données est relié ou est apte à être relié à un dispositif (44) de stockage de données au moyen d'une deuxième liaison de données, le premier signal audio (10) étant transmis par le dispositif (44) de stockage de données au dispositif (40) de traitement de données par l'intermédiaire de la deuxième liaison de données.

13. Procédé selon la revendication 11 ou la revendication 12, **caractérisé en ce que** la première connexion de données et/ou la deuxième connexion de données est ou sont ou seront établies via un réseau de données (42), en particulier via l'Internet.

14. Produit de programme informatique comprenant des moyens formant code de programme adaptés pour mettre en oeuvre un procédé selon l'une des revendications 1 à 13 lorsque les moyens formant code de programme sont exécutés sur un dispositif (40) de traitement de données.

15. Système informatique comprenant un dispositif (40) de traitement de données adapté pour mettre en oeuvre un procédé selon l'une quelconque des revendications 1 à 13.
